Europäisches Patentamt

European Patent Office

Office européen des brevets

(11) Veröffentlichungsnummer: **0 318 973**
**A2**

(12) **EUROPÄISCHE PATENTANMELDUNG**

(21) Anmeldenummer: 88120006.7

(51) Int. Cl.4: **G01N 33/00**

(22) Anmeldetag: 30.11.88

(30) Priorität: 03.12.87 DE 3741010

(43) Veröffentlichungstag der Anmeldung:
07.06.89 Patentblatt 89/23

(84) Benannte Vertragsstaaten:
DE FR GB IT NL

(71) Anmelder: Siemens Aktiengesellschaft Berlin
und München
Wittelsbacherplatz 2
D-8000 München 2(DE)

(72) Erfinder: Lange, Eckhard, Dipl.-Ing.
Siedlungstrasse 1
D-8192 Geretsried(DE)
Erfinder: Müller, Rudolf, Prof. Dr.
Ahornweg 22
D-8135 Söcking(DE)

(54) Sensoranordnung zur Flüssigkeitsanalyse mit mindestens zwei Gassensoren und Verfahren zum Betrieb einer Sensoranordnung zur Flüssigkeitsanalyse mit mindestens zwei Gassensoren.

(57) Gassensoren sind in einem Gehäuse in mindestens zwei Gruppen (9, 10) angeordnet. Gas kann - entweder dauernd oder alternierend - zunächst über die eine (9) und dann über die andere Gruppe (10) geleitet werden, wobei es zwischen den beiden Gruppen (9, 10) an einer mit einer Membran (8) verschlossenen Öffnung (7) vorbeiströmt. Die Membran (8) ist gasdurchlässig und flüssigkeitsundurchlässig. Das Gehäuse wird mit der Membran (8) in eine zu untersuchende Flüssigkeit getaucht. Das Gas strömt an der ersten Gruppe (9) vorbei und wird analysiert. An der Membran (8) entsteht ein Gemisch aus Gas und Flüssigkeitsdampf, das an der zweiten Gruppe (10) vorbeiströmt und analysiert wird. Durch Vergleich der Signale mit bekannten Mustern wird die Zusammensetzung des Dampfes und damit der Flüssigkeit bestimmt, wobei Einflüsse des Gases kompensiert werden.

FIG 1

## Sensoranordnung zur Flüssigkeitsanalyse mit mindestens zwei Gassensoren und Verfahren zum Betrieb einer Sensoranordnung zur Flüssigkeitsanalyse mit mindestens zwei Gassensoren

Die Erfindung betrifft eine Sensoranordnung zur Flüssigkeitsanalyse mit mindestens zwei Gassensoren in einem Gehäuse und ein Verfahren zur Flüssigkeitsanalyse mit einer Sensoranordnung in einem Gehäuse.

Es ist beispielsweise bei der großtechnischen Fermentation von Alkohol zu Essig wünschenswert, den Grad der Umwandlung in situ zu überwachen. Ferner ist es wünschenswert, die Konzentration von z. B. Lösungsmitteln im Abwasser von z. B. der chemischen Industrie zu überwachen.

Es ist bekannt (siehe z. B. M. Matsumura, H. Märkl, Biotechnologie and Bioengineering, Vol. 28 (1986), S. 534 ff, Gore-Tex Information der Fa. Gore), mit Hilfe einer Membran eine Phasentrennung vorzunehmen. Dabei wird ein Hohlkörper, der eine mit einer Membran verschlossene Öffnung aufweist, in eine Flüssigkeit getaucht. Die Membran ist gasdurchlässig und flüssigkeitsundurchlässig. Der Hohlkörper wird so in die Flüssigkeit getaucht, daß die Membran vollständig eingetaucht ist. Da die Membran gasdurchlässig aber flüssigkeitsundurchlässig ist, gelangen Teile der Flüssigkeit als Dampf in den Hohlkörper. Die Flüssigkeit kann hingegen nicht in den Hohlkörper gelangen. In dem Hohlkörper ist ein Gassensor vorgesehen, mit dem der Dampf analysiert wird.

Diese Vorrichtung ist, da sie nur einen Gassensor enthält, nur für wenige Gase empfindlich. Es ist damit nicht möglich, z. B. Essig und Alkohol zu unterscheiden. Ferner kommt es bei der Messung durch langsame Veränderung des Gases im Hohlkörper, z. B. der Umgebungsluft, zu Verfälschungen der Messungen.

Der Erfindung liegt die Aufgabe zugrunde, eine Anordnung und ein Verfahren zur Flüssigkeitsanalyse mit Hilfe von Gassensoren anzugeben, mit denen eine Vielzahl von Flüssigkeiten analysiert werden kann und mit denen insbesondere Einflüsse der Umgebung auf das Meßergebnis reduziert werden.

Die Aufgabe wird erfindungsgemäß mit einer Sensoranordnung zur Flüssigkeitsanalyse gelöst, wie dies im Anspruch 1 angegeben ist. Da in der Sensoranordnung eine Vielzahl von Gassensoren betrieben wird, ist die Empfindlichkeit gegenüber dem Stand der Technik deutlich erhöht. Durch Voranalyse des Trägergases, z. B. der Umgebungsluft, lassen sich Umwelteinflüsse auf das Meßergebnis kompensieren.

Die Sensoranordnung nach Anspruch 2 hat den Vorteil einer höheren Empfindlichkeit durch die Verwendung unterschiedlich gasdurchlässiger Membranen.

Die Sensoranordnung nach Anspruch 5 hat den Vorzug, daß das Kompensieren der Umwelteinflüsse besonders einfach ist.

Die Sensoranordnung nach Anspruch 7 ist zum dynamischen Betrieb geeignet. Gassensoren sind im dynamischen Betrieb wesentlich empfindlicher auf verschiedene Gase und Konzentrationen als im stationären Betrieb.

Die Sensoranordnung nach Anspruch 8 erlaubt wiederum eine einfache Kompensation der Umwelteinflüsse.

Die Sensoranordnung nach Anspruch 9 hat den Vorteil, daß die Information aus der Anlagerungsphase und diejenige aus der Ablagerungsphase gewonnen werden können.

Die Aufgabe wird weiterhin durch ein Verfahren zur Flüssigkeitsanalyse nach Anspruch 10 gelöst.

Die Verfahren nach Anspruch 11 und Anspruch 13 beinhalten dynamische Messungen der Gassensoren. Daher haben sie den Vorzug einer höheren Empfindlichkeit sowie des Gewinns weiterer Informationen gegenüber Verfahren, die stationäre Messungen verwenden.

Weitere Ausgestaltungen der Erfindung gehen aus den übrigen Ansprüchen hervor.

Ausführungsbeispiele der Erfindung sind in den FIG dargestellt und werden im folgenden näher erläutert.

FIG 1 stellt eine Sensoranordnung zur Flüssigkeitsanalyse dar, in der die Gassensoren in zwei Gruppen angeordnet sind.

FIG 2 stellt eine Sensoranordnung zur Flüssigkeitsanalyse dar, in der mehrere Anordnungen nach FIG 1 parallel betrieben werden.

FIG 3 stellt eine Sensoranordnung dar, in der die Gassensoren in drei Gruppen angeordnet sind.

FIG 4 stellt eine weitere Sensoranordnung dar, in der die Gassensoren in drei Gruppen angeordnet sind.

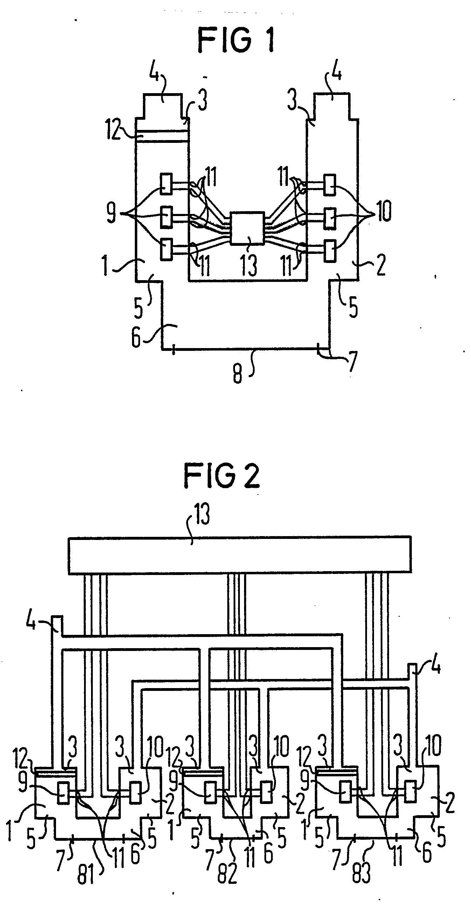

Die Sensoranordnung in FIG 1 weist ein erstes Rohrteil 1 und ein zweites Rohrteil 2 auf. An einer ersten Seite 3 sind beide Rohrteile mit je einem Gasanschluß 4 versehen. An einer zweiten Seite 5 sind die Rohrteile mit einem Zwischenbereich 6 verbunden. Der Zwischenbereich 6 weist eine Öffnung 7 auf, die durch eine Membran 8 verschlossen ist. Die Membran 8 hat die Eigenschaft, gasdurchlässig zu sein und dabei flüssigkeitsundurchlässig zu sein. Die Membran 8 ist daher zur Phasentrennung geeignet. Es kann eine handelsübliche Membran verwendet werden. In dem ersten Rohrteil 1 ist eine erste Gruppe Gassensoren 9 angeordnet.

In dem zweiten Rohrteil 2 ist eine zweite Gruppe Gassensoren 10 angeordnet. Es ist vorteilhaft, die erste Gruppe Gassensoren 9 und die zweite Gruppe Gassensoren 10 so anzuordnen, daß ein Gasstrom jeweils gleichzeitig an allen Sensoren der ersten Gruppe Gassensoren 9 bzw. der zweiten Gruppe Gassensoren 10 vorbeigeleitet werden kann. Beide Rohrteile weisen Durchführungen 11 für die elektrischen Anschlüsse der Gassensoren auf. Im ersten Rohrteil 1 ist eine Gastransportvorrichtung 12 vorgesehen. Die Gastransportvorrichtung 12 ist z. B. ein Ventilator.

Zur Flüssigkeitsanalyse wird die Sensoranordnung mit dem Zwischenbereich 6 in eine Flüssigkeit getaucht. Die Membran 8 ist dabei vollständig in die Flüssigkeit eingetaucht. Da die Membran 8 gasdurchlässig ist, gelangt der Dampf der Flüssigkeit durch die Membran 8 in den Zwischenbereich 6. Da die Membran 8 flüssigkeitsundurchlässig ist, kann die Flüssigkeit nicht in den Zwischenbereich 6 gelangen. Zur Messung wird durch den Gasanschluß 4 des ersten Rohrteils 1 Gas in das erste Rohrteil 1 eingeleitet. Das geschieht z. B. durch Anschluß des Gasanschlußes 4 an eine Gasflasche, die unter Überdruck steht. Eine weitere Möglichkeit ist, durch den Gasanschluß 4 des ersten Rohrteils 1 mit Hilfe der Gastransportvorrichtung 12 Umgebungsluft anzusaugen. Das Gas wird von der ersten Gruppe Gassensoren 9 analysiert. Im Zwischenbereich 6 vermischt sich das Gas mit dem Dampf der Flüssigkeit. Dieses Gasgemisch strömt weiter in das zweite Rohrteil 2. Dort wird es von der zweiten Gruppe Gassensoren 10 analysiert. Durch den Gasanschluß 4 des zweiten Rohrteils 2 verläßt das Gasgemisch die Sensoranordnung. Die gemessenen Signale werden in einer Mustererkennungsmatrix 13, wie sie z. B. aus DE 35 19 436 A1, DE 35 19 435 A1, DE 35 19 410 A1 oder DE 35 19 397 A1 bekannt ist, mit bekannten Mustern verglichen. Durch die Signale der ersten Gruppe Gassensoren 9, die nur Information über das Gas enthalten, läßt sich der Einfluß des Gases auf die Signale der zweiten Gruppe Gassensoren 10 kompensieren. Dadurch läßt sich die Zusammensetzung des Dampfes der Flüssigkeit bestimmen. Damit erhält man Informationen über die Zusammensetzung der Flüssigkeit.

Die Berücksichtigung des Gases ist besonders einfach, wenn es zu jedem Gassensor der ersten Gruppe Gassensoren 9 in der zweiten Gruppe Gassensoren 10 einen Gassensor gleicher Bauart gibt. Dabei ist es empfehlenswert, solche Gassensorpaare auszuwählen, deren Eigenschaften möglichst wenig streuen.

Eine weitere Betriebsart der Sensoranordnung besteht darin, den Gasstrom nach jeder Messung umzudrehen. Dabei wird das Gas von der Gastransportvorrichtung 12 bei der ersten Messung vom ersten Rohrteil 1 ins zweite Rohrteil 2 geleitet, bei der nächsten Messung wird das Gas vom zweiten Rohrteil 2 zum ersten Rohrteil 1 geleitet. Diese Betriebsart hat den Vorteil, daß eine dynamische Messung möglich ist. Während der Anlagerung eines Gases an einen Gassensor ist die Signalhöhe und Signalart anders als in der stationären Phase, in der An- und Ablagerung des Gases im Gleichgewicht sind. Dieses Anlaufverhalten ist abhängig von Gasart und Gaskonzentration. Durch Messung des dynamischen Verhaltens eines Gassensors erhält man andere und deswegen zusätzliche Informationen als nur durch Messung im stationären Betrieb des Gassensors. Durch Veränderung der Strömungsrichtung des Gases in der Sensoranordnung wird die Rolle der zwei Gruppen Gassensoren bei jeder Messung umgekehrt. Bei der ersten Messung ist die erste Gruppe Gassensoren 9 z. B. für die Voranalyse des Gases zuständig, während die zweite Gruppe Gassensoren 10 für die Analyse des Gasgemisches zuständig ist. In der zweiten Messung ist die erste Gruppe Gassensoren 9 für die Analyse des Gemisches zuständig. Dabei wird die Anlagerungsphase des Dampfes an die Gassensoren der ersten Gruppe Gassensoren 9 registriert. Bei den Sensoren der zweiten Gruppe Gassensoren 10, die in dieser Messung für die Voranalyse des Gases zuständig sind, findet zunächst nur die Ablagerung des Dampfes statt, wodurch wiederum weitere Informationen gewonnen werden können. Danach dienen die Sensoren der zweiten Gruppe Gassensoren 10 zur Analyse des Gases.

In FIG 2 ist eine Sensoranordnung zur Flüssigkeitsanalyse dargestellt, die mehrere Sensoranordnungen nach dem ersten Ausführungsbeispiel enthält. Es sind z. B. drei Sensoranordnungen vorgesehen. Diese Sensoranordnungen werden in dem Sinne parallel betrieben, daß die Gasanschlüsse 4, über die das Gas zuströmt, und die Gasanschlüsse 4, über die das Gas abströmt, zusammengefaßt sind. Jede der Sensoranordnungen weist das erste Rohrteil 1 und das zweite Rohrteil 2 auf. An den ersten Seiten 3 sind die Rohrteile mit den Gasanschlüssen 4 verbunden. An den zweiten Seiten 5 sind jeweils das erste Rohrteil 1 und das zweite Rohrteil 2 über den Zwischenbereich 6 verbunden. Im ersten Rohrteil 1 ist die erste Gruppe Gassensoren 9, im zweiten Rohrteil 2 die zweite Gruppe Gassensoren 10 angeordnet. Die erste Gruppe Gassensoren 9 und die zweite Gruppe Gassensoren 10 bestehen z. B. jeweils aus einem Gassensor. Die elektrischen Anschlüsse der Gassensoren werden über die Durchführungen 11 aus dem Gehäuse geführt und mit der Mustererkennungsmatrix 13 verbunden. Die Zwischenbereiche 6 weisen jeweils die Öffnung 7 auf. In der ersten Sensoranordnung ist die Öffnung 7 mit einer ersten Membran 81 verschlossen. In der zweiten Sensoranordnung ist die Öffnung 7 mit einer zweiten Membran 82 verschlossen. In der dritten Sensoranordnung ist die Öffnung 7 mit einer dritten Membran 83 verschlossen. Die erste Membran 81, die zweite Membran 82 und die dritte Membran 83 sind flüssigkeitsundurchlässig und gasdurchlässig. Sie unterscheiden sich in ihrer Gasdurchlässigkeit hinsichtlich der durchgelassenen Gase. Diese Sensoranordnung bietet daher den Vorteil, eine höhere Selektivität zu haben und damit besser zwischen verschiedenen Flüssigkeiten unterscheiden zu können.

Das Betriebsverfahren für die in FIG 2 dargestellte Sensoranordnung ist analog den für die in FIG 1 dargestellte Sensoranordnung beschriebenen Betriebsverfahren.

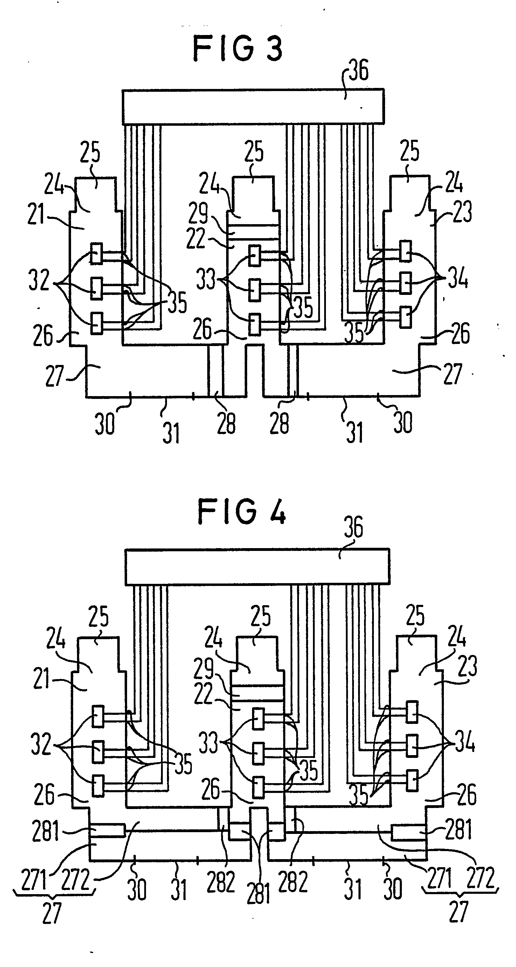

In FIG 3 ist ein weiteres Ausführungsbeispiel der Erfindung dargestellt. Es ist ein weiteres erstes Rohrteil 21, ein weiteres zweites Rohrteil 22 und ein weiteres drittes Rohrteil 23 vorgesehen. Die Rohrteile weisen an einer weiteren ersten Seite 24 je einen weiteren Gasanschluß 25 auf. Das weitere erste Rohrteil 21 und das weitere zweite Rohrteil 22 sowie das weitere zweite Rohrteil 22 und das weitere dritte Rohrteil 23 sind an einer weiteren zweiten Seite 26 über je einen weiteren Zwischenbereich 27 verbunden. Die weiteren Zwischenbereiche 27 weisen je ein Ventil 28 auf. Ferner weisen die weiteren Zwischenbereiche 27 je eine weitere Öffnung 30 auf, die durch je eine weitere Membran 31 verschlossen ist. Die weiteren Membranen 31 haben die Eigenschaft, gasdurchlässig und dabei flüssigkeitsundurchlässig zu sein. Die weiteren Membranen 31 sind daher zur Phasentrennung geeignet. Es werden handelsübliche Membranen verwendet.

Im weiteren zweiten Rohrteil 22 ist eine weitere Gastransportvorrichtung 29 vorgesehen. In dem weiteren ersten Rohrteil 21 ist eine weitere erste Gruppe Gassensoren 32 angeordnet. In dem weiteren zweiten Rohrteil 22 ist eine weitere zweite Gruppe Gassensoren 33 angeordnet. In dem weiteren dritten Rohrteil 23 ist eine dritte Gruppe Gassensoren 34 angeordnet. Die Rohrteile weisen weitere Durchführungen 35 für die elektrischen Anschlüsse der Gassensoren auf.

Zur Flüssigkeitsanalyse wird die Sensoranordnung mit den weiteren Zwischenbereichen 27 in eine Flüssigkeit getaucht. Die weiteren Membranen 31 sind dabei vollständig in die Flüssigkeit eingetaucht. Da die weiteren Membranen 31 gasdurchlässig sind, gelangt der Dampf der Flüssigkeit durch die weiteren Membranen 31 in die weiteren Zwischenbereiche 27. Da die weiteren Membranen 31 flüssigkeitsundurchlässig sind, kann die Flüssigkeit nicht in die weiteren Zwischenbereiche 27 gelangen.

Zum Betrieb der Sensoranordnung wird der weitere Gasanschluß 25 des weiteren zweiten Rohrteils 22 z. B. an eine Gasflasche, die unter Überdruck steht, angeschlossen. Eine weitere Möglichkeit ist, durch den weiteren Gasanschluß 25 des weiteren zweiten Rohrteils 22 Raumluft anzusaugen. Es ist vorteilhaft, die weiteren Gasanschlüsse 25 des weiteren ersten Rohrteils 21 und des weiteren dritten Rohrteils 23 mit einer Abluftentsorgung zu verbinden. Mit der weiteren Gastransportvorrichtung 29 wird das Gas in das weitere zweite Rohrteil 22 geleitet. Die weitere Gastransportvorrichtung 29 ist z. B. ein Ventilator. Das Gas wird von den Gassensoren der weiteren zweiten Gruppe Gassensoren 33 voranalysiert. Das Ventil 28, das in dem weiteren Zwischenbereich 27 vorgesehen ist, der die Verbindung zum weiteren dritten Rohrteil 23 gewährleistet, ist verschlossen. Das Ventil 28, das in der Verbindung zum weiteren ersten Rohrteil 21 angebracht ist, ist offen. Daher strömt das Gas durch den weiteren Zwischenbereich 27 zum weiteren ersten Rohrteil 21. Die weitere Membran 31 ist gasdurchlässig und flüssigkeitsundurchlässig. Durch die weitere Membran 31 gelangt Dampf der Flüssigkeit in den weiteren Zwischenbereich 27. Im weiteren Zwischenbereich 27 mischt sich der Dampf der Flüssigkeit mit dem eingeleiteten Gas. Die Gasströmung führt das entstandene Gemisch an der weiteren ersten Gruppe Gassensoren 32 vorbei. Das Gasgemisch wird von der weiteren ersten Gruppe Gassensoren 32 analysiert. Als Meßgröße wird das Anlaufverhalten der Gassensoren der weiteren ersten Gruppe 32 registriert. Während der Anlaufphase eines Gassensors ist die Signalhöhe im wesentlichen durch Anlagerungsprozesse des Gases an den Gassensor bestimmt. Im stationären Betrieb eines Gassensors wird die Signalhöhe dagegen von im Gleichgewicht zueinander befindlichen Anlagerungs- und Ablagerungsprozessen bestimmt. Die Anlagerungsprozesse sind stark von der Gasart und der Gaskonzentration abhängig.

Für die nächste Messung wird das Ventil 28 in der Verbindung zum weiteren ersten Rohrteil 21 geschlossen und das Ventil 28 in der Verbindung zum weiteren dritten Rohrteil 23 geöffnet. Das Gas strömt nun vom weiteren zweiten Rohrteil 22 zum weiteren dritten Rohrteil 23. Dabei wird das Gas von den Gassensoren der weiteren zweiten Gruppe Gassensoren 33 voranalysiert. Im weiteren Zwischenbereich 27 findet eine Vermischung des Gases mit dem durch die Membran in den Zwischenbereich 27 gelangten Dampf statt. Das Gasgemisch wird von den Sensoren der dritten Gruppe Gassensoren 34 im weiteren dritten Rohrteil 23 analysiert. Wiederum wird die Anlaufphase der Gassensoren registriert. Durch Vergleich der erhaltenen Meßdaten in einer weiteren Mustererkennungsmatrix 36, wie sie aus DE 35 19 436 A1, DE 35 19 435 A1, DE 35 19 410 A1 und DE 35 19 397 A1 bekannt ist, mit bekannten Mustern läßt sich die Zusammensetzung des Dampfes und damit der Flüssigkeit ermitteln. Der Einfluß des Gases, mit dem die Strömung durch den Gassensor realisiert wird und das nichts mit der zu analysierenden Flüssigkeit zu tun hat, wird durch die Meßwerte, die im weiteren zweiten Rohrteil 22 mit den Sensoren der weiteren zweiten Gruppe Gassensoren 33 erhalten werden, kompensiert.

Für eine weitere Messung wird das Ventil 28 in der Verbindung zum weiteren dritten Rohrteil 23 geschlossen und das Ventil 28 in der Verbindung zum weiteren ersten Rohrteil 21 wieder geöffnet. Nun wird das Gasgemisch wiederum von den Gassensoren der weiteren ersten Gruppe Gassensoren 32 analysiert. Damit wiederum die Anlaufphase der Gassensoren der weiteren ersten Gruppe Gassensoren 32 beobachtet werden kann, muß die Zeit zwischen zwei Messungen so bemessen sein, daß das an die Gassensoren angelagerte Gas sich wieder abgelagert hat. Typische Zeiten für Ablagerungsprozesse sind Sekunden bis Minuten. Diese Zeiten können durch eine kurzzeitige Erhöhung der Betriebstemperatur der Sensoren verkürzt werden.

In FIG 4 ist eine Sensoranordnung dargestellt, die sich von der Sensoranordnung aus FIG 3 durch die Ausgestaltung der weiteren Zwischenbereiche 27 unterscheidet. Alle übrigen Teile der Sensoranordnung sind identisch mit denen aus FIG 3. Die weiteren Zwischenbereiche 27 enthalten je einen ersten Kanal 271 und einen zweiten Kanal 272. Die ersten Kanäle 271 und die zweiten Kanäle 272 verlaufen jeweils vom weiteren zweiten Rohrteil 22 zum weiteren ersten Rohrteil 21 bzw. zum weiteren dritten Rohrteil 23. Die ersten Kanäle 271 sind so angeordnet, daß sie jeweils die mit der weiteren Membran 31 verschlossene weitere Öffnung 30 enthalten. In den ersten Kanälen 271 sind je zwei erste Ventile 281 vorgesehen, mit denen die ersten Kanäle 271 diesseits und jenseits der weiteren Öffnungen 30 abgesperrt werden können. Die zweiten Kanäle 272 sind so angeordnet, daß sie die weiteren Öffnungen 30 jeweils nicht enthalten. In den zweiten Kanälen 272 ist jeweils ein zweites Ventil 282 vorgesehen, mit dem die zweiten Kanäle 272 abgesperrt werden können. Die ersten Kanäle 271 und die zweiten Kanäle 272 sind so zueinander angeordnet, daß ein Gasstrom bei verschlossenen ersten Ventilen 281 und offenem zweiten Ventil 282 nur jeweils durch den zweiten Kanal 272 und bei verschlossenem zweiten Ventil 282 und offenen ersten Ventilen 281 nur jeweils durch den ersten Kanal 271 strömt.

Im Betrieb der Sensoranordnung nach FIG 4 sind die ersten Ventile 281 und die zweiten Ventile 282 jeweils so geschaltet, daß der Gasstrom in dem einen weiteren Zwischenbereich 27 durch den ersten Kanal 271 strömt, während der Gasstrom in dem anderen weiteren Zwischenbereich 27 durch den zweiten Kanal 272 strömt. In dem weiteren Zwischenbereich 27, in dem der Gasstrom durch den ersten Kanal 271 strömt, kommt es zu einer Vermischung des Gases mit dem Flüssigkeitsdampf. In dem weiteren Zwischenbereich 27, in dem der Gasstrom durch den zweiten Kanal 272 strömt, kommt das Gas nicht mit dem Flüssigkeitsdampf in Berührung; es findet keine Vermischung statt. Diejenige Gruppe Gassensoren, an der das Gasgemisch vorbeiströmt, analysiert das Gasgemisch. Es findet eine Anlagerung des Gasgemisches und damit des Flüssigkeitsdampfes an die Gassensoren statt. Die Parameter der Anlagerungsphase werden registriert. Diejenige Gruppe Gassensoren, an der das unvermischte Gas vorbeiströmt, analysiert lediglich das unvermischte Gas. Bei der ersten Messung liefern diese Gassensoren keine zusätzliche Information. Für die nächste Messung werden die ersten Ventile 281 und die zweiten Ventile 282 umgeschaltet, so daß der Gasstrom nun durch den jeweils anderen Kanal des weiteren Zwischenbereichs 27 strömt. Dadurch gelangt das Gemisch aus Gas und Flüssigkeitsdampf zu derjenigen Gruppe Gassensoren, die bei der vorherigen Messung das reine Gas analysiert haben. An diese Gassensoren findet die Anlagerung des Gasgemisches statt. Die Parameter der Anlagerungsphase werden aufgenommen. Diejenige Gruppe Gassensoren, die in der vorigen Messung das Gasgemisch analysiert haben, sehen in dieser Messung das reine Gas. Es findet die Ablagerung des Flüssigkeitsdampfes von den Gassensoren statt. Die Parameter der Ablagerung werden registriert. Für die wiederum nächste Messung werden die ersten Ventile 281 und die zweiten Ventile 282 wiederum umgeschaltet. Bei jeder folgenden Messung werden von einer Gruppe Gassensoren die Parameter der Anlagerung des Flüssigkeitsdampfes registriert, während von der anderen Gruppe Gassensoren die Parameter der Ablagerung des Flüssigkeitsdampfes registriert werden. Mit dieser Anordnung ist es möglich, alle Parameter im dynamischen Betrieb der Sensoranordnung zu messen. Diese Anordnung ist daher sehr empfindlich für verschiedene Flüssigkeiten. Der dynamische Betrieb ist nicht nur wegen der höheren Empfindlichkeit vorteilhaft, sondern auch weil er die Möglichkeit einer schnelleren Messung bietet. Es muß nicht jedes Mal abgewartet werden, bis sich der stationäre Betrieb eingestellt hat.

Als Gassensoren können alle bekannten Gassensoren verwendet werden. Da die Auswertung über Mustervergleich in der weiteren Mustererkennungsmatrix 36 erfolgt, ist es günstig, Gassensoren mit nicht nur Haupt- sondern auch Querempfindlichkeiten zu verwenden.

Es ist möglich, die Gassensoren einer Temperaturmodulation zu unterziehen, um die Empfindlichkeitsbereiche der Sensoren zu erweitern, wie aus z. B. DE 35 19 410 A1 bekannt. Dabei ist es vorteilhaft, den Gasstrom periodisch umzuschalten. Wird der Gasstrom mit einer Frequenz f1, die verschieden von der Frequenz f2 der Temperaturmodulation ist, umgeschaltet, können im Frequenzspektrum bei der Summe der Frequenzen f1 + f2 muliplikative Verknüpfungen analysiert werden.

**Ansprüche**

1. Sensoranordnung zur Flüssigkeitsanalyse mit mindestens zwei Gassensoren in einem Gehäuse mit folgenden Merkmalen:
   a) das Gehäuse weist mindestens einen Zwischenbereich (6), einen Gaseinlaß (4) und einen Gasauslaß (4) auf,
   b) die Gassensoren sind mindestens in eine erste Gruppe (9) und eine zweite Gruppe (10) aufgeteilt,
   c) die Gassensoren sind so angeordnet, daß Gas von dem Gaseinlaß (4) her an mindestens einem Sensor der ersten Gruppe Gassensoren (9) vorbei strömt, daß es durch den Zwischenbereich (6) strömt und daß es an mindestens einem Sensor der zweiten Gruppe Gassensoren (10) vorbei zu dem Gasauslaß (14) strömt,
   d) der Zwischenbereich (6) enthält eine Membran (8), die gasdurchlässig und flüssigkeitsundurchlässig ist und die daher zur Phasentrennung geeignet ist.

2. Sensoranordnung nach Anspruch 1, **gekennzeichnet** durch folgende Merkmale:
   a) mindestens zwei Sensoranordnungen, die eine erste Gruppe (9) und eine zweite Gruppe (10) Gassensoren und einen Zwischenbereich (6) enthalten, sind zwischen Gaseinlaß (4) und Gasauslaß (4) parallel geschaltet,
   b) jeder Zwischenbereich (6) weist eine Membran (81, 82, 83) auf, die gasdurchlässig und flüssigkeitsundurchlässig ist,
   c) die Membranen (81, 82, 83) sind untereinander bezüglich der Gasdurchlässigkeit verschieden.

3. Sensoranordnung nach Anspruch 1 oder 2, **dadurch gekennzeichnet,** daß eine Gastransportvorrichtung (12) vorgesehen ist, mit der Gas sowohl vom Gaseinlaß (4) zum Gasauslaß (4) als auch vom Gasauslaß (4) zum Gaseinlaß (4) geleitet werden kann.

4. Sensoranordnung nach einem der Ansprüche 1 bis 3, **gekennzeichnet** durch folgende Merkmale:
   a) das Gehäuse weist mindestens zwei Rohrteile (1, 2) auf,
   b) jedes Rohrteil (1, 2) ist mit einem Gasanschluß (4) verbunden,
   c) in jedem Rohrteil (1, 2) ist mindestens ein Sensor einer Gruppe Gassensoren (9, 10) angeordnet,
   d) an der dem Gasanschluß abgewandten Seite sind die Rohrteile (1, 2) mit dem Zwischenbereich (6) verbunden,
   e) an jedem Rohrteil (1, 2) sind Durchführungen (11) für die elektrischen Anschlüsse der Gassensoren vorgesehen.

5. Sensoranordnung nach einem der Ansprüche 1 bis 4, dadurch **gekennzeichnet,** daß der ersten Gruppe Gassensoren (9) ebenso viele Gassensoren angehören wie der zweiten Gruppe (10) und daß es zu jedem Gassensor in der ersten Gruppe (9) einen Gassensor gleicher Bauart in der zweiten Gruppe (10) gibt.

6. Sensoranordnung nach Anspruch 1, **gekennzeichnet** durch folgende Merkmale:
   a) das Gehäuse weist einen weiteren Zwischenbereich (27) und einen weiteren Gasauslaß (25) auf,
   b) die Gassensoren sind in drei Gruppen so angeordnet, daß Gas vom Gaseinlaß (25) an der zweiten Gruppe Gassensoren (33) vorbeiströmt, daß es durch die Zwischenbereiche (27) strömt und daß es an der ersten Gruppe Gassensoren (32) und der dritten Gruppe Gassensoren (34) vorbei zu den Gasauslassen (25) strömt,
   c) in den Zwischenbereichen (27) ist je mindestens ein Ventil (28) vorgesehen, mit dem der Zwischenbereich (27) abgesperrt werden kann,
   d) der weitere Zwischenbereich (27) weist eine weitere Membran (31) auf, die gasdurchlässig und flüssigkeitsundurchlässig ist.

7. Sensoranordnung nach Anspruch 4, **gekennzeichnet** durch folgende Merkmale:
   a) das Gehäuse weist ein weiteres Rohrteil (23) auf,
   b) das weitere Rohrteil (23) ist direkt mit einem weiteren Gasanschluß (25) verbunden,
   c) in dem weiteren Rohrteil (23) ist eine dritte Gruppe Gassensoren (34) angeordnet,
   d) an der dem Gasanschluß (25) abgewandten Seite (26) ist das weitere Rohrteil (23) über einen weiteren Zwischenbereich (27) mit einem der Rohrteile (22) verbunden,
   e) der weitere Zwischenbereich (27) weist eine weitere Membran (31) auf, die gasdurchlässig und flüssigkeitsundurchlässig ist und die daher zur Phasentrennung geeignet ist,

f) das weitere Rohrteil (23) weist weitere Durchführungen (35) für die elektrischen Anschlüsse auf,

g) der Zwischenbereich (27) und der weitere Zwischenbereich (27) sind mit je mindestens einem Ventil (28) versehen, mit denen der Bereich jeweils abgesperrt werden kann.

8. Sensoranordnung nach Anspruch 6 oder 7, **gekennzeichnet** durch folgende Merkmale:

a) der ersten Gruppe Gassensoren (32) gehören ebenso viele Gassensoren an wie der zweiten Gruppe (33),

b) der ersten Gruppe Gassensoren (32) gehören ebenso viele Gassensoren an wie der dritten Gruppe (34),

c) zu jedem Gassensor in der ersten Gruppe (32) gibt es je einen Gassensor gleicher Bauart in der zweiten Gruppe (33) und der dritten Gruppe (34).

9. Sensoranordnung nach einem der Ansprüche 6 bis 8, **gekennzeichnet durch** folgende Merkmale:

a) die Zwischenbereiche (27) sind je in einen ersten Kanal (271) und einen zweiten Kanal (272) unterteilt,

b) in jedem Kanal (271, 272) ist mindestens ein Ventil (281, 282) vorgesehen, mit dem der Kanal (271, 272) abgesperrt werden kann,

c) der erste Kanal (271) enthält jeweils vollständig die Membran (31) und ist vor und hinter der Membran (31) durch Ventile (281) absperrbar.

10. Verfahren zur Flüssigkeitsanalyse mit einer Sensoranordnung nach Anspruch 4 oder 5, mit folgenden Schritten:

a) die Sensoranordnung wird mit dem Zwischenbereich (6) in eine zu untersuchende Flüssigkeit getaucht, so daß die Membran (8) ganz bedeckt ist, dabei gelangt Flüssigkeitsdampf in den Zwischenbereich (6) und es findet eine Phasentrennung durch die Membran (8) statt,

b) Gas wird in das erste Rohrteil (1) eingeleitet,

c) das Gas wird von den im ersten Rohrteil (1) befindlichen Sensoren analysiert,

d) das Gemisch aus Gas und Flüssigkeitsdampf, das sich im Zwischenbereich (6) gebildet hat und das durch das zweite Rohrteil (2) strömt, wird von den im zweiten Rohrteil (2) befindlichen Sensoren analysiert,

e) die Signale der Sensoren werden in einer Mustererkennungsmatrix (13) mit bekannten Mustern verglichen und damit wird über den Flüssigkeitsdampf die Flüssigkeit bestimmt.

11. Verfahren nach Anspruch 10, dadurch **gekennzeichnet,** daß nach jeder Messung die Richtung des Gasstroms umgekehrt wird, wobei die Information aus der Anlagerungsphase und aus der Ablagerungsphase registriert wird.

12. Verfahren nach Anspruch 11, dadurch **gekennzeichnet,** daß die Gassensoren temperaturmoduliert betrieben werden, wobei die Frequenz der Temperaturmodulation verschieden ist von der Frequenz der Richtungsumkehr des Gasstromes.

13. Verfahren zur Flüssigkeitsanalyse mit einer Sensoranordnung nach einem der Ansprüche 7 bis 9 mit folgenden Schritten:

a) die Sensoranordnung wird mit den Zwischenbereichen (27) in eine zu untersuchende Flüssigkeit getaucht, so daß die Membranen (31) ganz bedeckt sind, dabei findet eine Phasentrennung durch die Membranen (31) statt und es gelangt Flüssigkeitsdampf in die Zwischenbereiche (27),

b) mit der Gastransportvorrichtung (29) wird Gas in das zweite Rohrteil (22) eingeleitet,

c) das Gas wird von den im zweiten Rohrteil (22) befindlichen Sensoren analysiert,

d) das Gemisch aus Gas und Flüssigkeitsdampf, das sich in den Zwischenbereichen (27) gebildet hat und das je nach Ventilstellung durch das erste Rohrteil (21) oder das dritte Rohrteil (23) strömt, wird von den im jeweiligen Rohrteil (21, 23) befindlichen Sensoren analysiert,

e) die Signale der Sensoren werden in einer Mustererkennungsmatrix (36) mit bekannten Mustern verglichen und damit wird über den Flüssigkeitsdampf die Flüssigkeit bestimmt,

f) bei der nächsten Messung wird das Gemisch aus Gas und Flüssigkeitsdampf durch Umschalten der Ventile (28) in das jeweils andere Rohrteil (23, 21) geleitet und analysiert.

14. Verfahren nach Anspruch 13, **gekennzeichnet** durch folgende Merkmale:

a) für die erste Messung werden die Ventile (281, 282) in den Zwischenbereichen (27) so geschaltet, daß der Gasstrom vom zweiten Rohrteil (22) zum ersten Rohrteil (21) durch den ersten Kanal (271) strömt und daß der Gasstrom vom zweiten Rohrteil (22) zum dritten Rohrteil (23) durch den zweiten Kanal (272) strömt,

b) für die zweite Messung werden die Ventile (281, 282) in den Zwischenbereichen (27) so geschaltet, daß der Gasstrom vom zweiten Rohrteil (22) zum ersten Rohrteil (21) durch den zweiten Kanal (272) strömt und daß der Gasstrom vom zweiten Rohrteil (22) zum dritten Rohrteil (23) durch den ersten Kanal (271) strömt,

c) die erste und die zweite Messung werden ständig abgewechselt.

# FIG 1

# FIG 2

EP 0 318 973 A2

FIG 3

FIG 4